(19) Europäisches Patentamt
European Patent Office
Office européen des brevets

(11) **EP 1 526 381 A1**

(12) **EUROPEAN PATENT APPLICATION**

(43) Date of publication:
**27.04.2005 Bulletin 2005/17**

(51) Int Cl.7: **G01N 33/574**

(21) Application number: **03380238.0**

(22) Date of filing: **21.10.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **Medplant Genetics S.L.**
**48160 Derio (Vizcaya) (ES)**

(72) Inventors:
- **Del Amo Iribarren, Jokin**
**48160 Derio, Vizcaya (ES)**
- **Junquera Sanchez-Vallejo, Corina**
**48160 Derio, Vizcaya (ES)**
- **Ochoa Garay, Jorge**
**48160 Derio, Vizcaya (ES)**
- **Escuredo Garcia de Galdeano, Pedro**
**48160 Derio, Vizcaya (ES)**
- **Santa Cruz, Simon**
**48160 Derio, Vizcaya (ES)**
- **Martinez Martinez, Antonio**
**48160 Derio, Vizcaya (ES)**
- **Simon Buela, Laureano**
**48160 Derio, Vizcaya (ES)**
- **Arguelles Sanchez, Maria Eladia**
**Principado, 3, 4o Oviedo (ES)**
- **De Los Toyos Gonzalez, Juan Ramon**
**Principado, 3, 4o Oviedo (ES)**
- **Barneo Serra, Luis**
**Principado, 3, 4o Oviedo (ES)**

(74) Representative: **Carpintero Lopez, Francisco**
**Herrero & Asociados, S.L.,**
**Alcalá, 35**
**28014 Madrid (ES)**

Remarks:
The sequence listing, which is published as annex to the application documents, was filed after the date of filing. The applicant has declared that it does not include matter which goes beyond the content of the application as filed.

(54) **Methods for in vitro diagnosis and in vitro prognosis of cancer of the pancreas and for the development of drugs against cancer of the pancreas**

(57) The present invention refers to an *in vitro* method to detect a cancer of the pancreas, especially a ductal adenocarcinoma of the pancreas, in an individual, to determine the stage or severity of this cancer in an individual or to monitor the effect of therapy administered to an individual with this cancer; to screen for, identify, develop and evaluate the efficacy of therapeutic compounds against this cancer in order to develop new medicinal products, and also agents that inhibit the expression and/or activity of the DDEF2 protein and/or the effects of this expression.

EP 1 526 381 A1

SEQUENCE LISTING

<110> Medplant Genetics S.L.

<120> METHODS FOR IN VITRO DIAGNOSIS AND IN VITRO PROGNOSIS OF CANCER OF THE PANCREAS AND FOR THE DEVELOPMENT OF DRUGS AGAINST CANCER OF THE PANCREAS

<130> 2003893

<140> EP 03380238.0
<141> 2003-10-21

<160> 15

<170> PatentIn version 3.1

<210> 1
<211> 20
<212> DNA
<213> Artificial

<220>
<221> misc_feature
<223> Primer for PCR amplification of the cDNA of DDEF2 gene

<400> 1
gcatgccatt ctccatcaga 20

<210> 2
<211> 22
<212> DNA
<213> Artificial

<220>
<221> misc_feature
<223> Primer for PCR amplification of the cDNA of DDEF2 gene

<400> 2
ccacccctta acagatcaca ca 22

<210> 3
<211> 25
<212> DNA
<213> Artificial

<220>
<221> misc_feature
<223> Probe corresponding to the set of probes 206414_s_at

<400> 3
ctccagtccc aaactaatct gtcag 25

<210> 4
<211> 25
<212> DNA
<213> Artificial

<220>
<221> misc_feature
<223> Probe correponding to the set of probes 206414_s_at

<400> 4
aatctgtcag gttcactggt acata 25

<210> 5

<211> 25
<212> DNA
<213> Artificial

<220>
<221> misc_feature
<223> Probe corresponding to the set of probes 206414_s_at

<400> 5
gaaattatat taacgaccct gctaa 25

<210> 6
<211> 25
<212> DNA
<213> Artificial

<220>
<221> misc_feature
<223> Probe corresponding to the set of probes 206414_s_at

<400> 6
gaccctgcta atatcctttc ttagt 25

<210> 7
<211> 25
<212> DNA
<213> Artificial

<220>
<221> misc_feature
<223> Probe corresponding to the set of probes 206414_s_at

<400> 7
ctttcttagt tatttgctcc ttgca 25

<210> 8
<211> 25
<212> DNA
<213> Artificial

<220>
<221> misc_feature
<223> Probe corresponding to the set of probes 206414_s_at

<400> 8
cagctgcagg attctggcat tttgc 25

<210> 9
<211> 25
<212> DNA
<213> Artificial

<220>
<221> misc_feature
<223> Probe corresponding to the set of probes 206414_s_at

<400> 9
ggcattttgc atgccattct ccatc 25

<210> 10
<211> 25
<212> DNA
<213> Artificial

<220>
<221> misc_feature
<223> Probe corresponding to the set of probes 206414_s_at

<400> 10
gatggctcag aacatgtaca cagac 25

<210> 11
<211> 25
<212> DNA
<213> Artificial

<220>
<221> misc_feature
<223> Probe corresponding to the set of probes 206414_s_at

<400> 11
gatattcagt tcattcacct gatta 25

<210> 12
<211> 25
<212> DNA
<213> Artificial

<220>
<221> misc_feature
<223> Probe corresponding to the set of probes 206414_s_at

<400> 12
cacctgatta ctttggttgc agcac 25

<210> 13
<211> 25
<212> DNA
<213> Artificial

<220>
<221> misc_feature
<223> Probe corresponding to the set of probes 206414_s_at

<400> 13
ttactcttct tgttaactag tcatt 25

<210> 14
<211> 17
<212> DNA
<213> Artificial

<220>
<221> misc_feature
<223> Primer for the PCR amplification of the gene coding for the human ribosomal protein L10

<400> 14
tgcgatggct gcacaca 17

<210> 15
<211> 23
<212> DNA
<213> Artificial

<220>
<221> misc_feature
<223> Primer for the PCR amplification of the gene coding for the

humanribosomal protein L10

<400> 15
tcccttagag caacccatac aac 23

## Description

### FIELD OF THE INVENTION

The present invention refers to an *in vitro* method to detect the presence of a cancer of the pancreas in an individual, to determine the stage or severity of this cancer in the individual, or to monitor the effect of therapy administered to an individual with the said cancer; to screen for, identify, develop and evaluate the efficacy of therapeutic compounds for this cancer in an attempt to develop new medicinal products and to agents that inhibit expression of the DDEF2 protein, and/or the effects of this expression.

### BACKGROUND OF THE INVENTION

Cancer of the pancreas was the cause of more than 220,000 deaths worldwide and more than 3,600 in Spain during the year 2,000 (GLOBOCAN). The clinical behaviour of pancreatic cancer is homogeneous and always unfavourable and there are no clear differences in survival in the different stages. Only very few patients with pancreatic cancer experience favourable outcomes. A possible explanation for this is that even in patients with small tumours, classified as stage I, the disease has spread beyond the pancreas. Its diagnosis in the initial stages, except for exceptional cases, is very difficult. Of patients diagnosed, 75% present the disease in advanced stages (Stages III and IV). It is a highly aggressive neoplasm resistant to cytostatic treatments and only between 1 and 4% of cases are alive five years after diagnosis corresponding to cases in which the tumour is localised and has been completely extirpated (Warshaw A.L., and Fernandes del Castillo C., N. Eng. J. Med., 1992, 326:455-465; Ahlgren J.D., Semin. Oncol., 1996, 23:241-250). For these reasons, the development of early diagnostic procedures and effective therapies are crucial to control this disease (Byungwoo R., et al., Cancer Res., 2002, 62:819-826).

Many of the genes and proteins involved in the development of the cancer of the pancreas are still unknown. Identification of genes and proteins expressed differentially in association with the invasive tumoral process could lead to the identification of biological markers and therapeutic targets that could be very valuable in the development of new tools for the diagnosis, prognosis and treatment of this disease as well as in the development of new therapeutic agents for treatment and/or prevention of the disease.

The Development and Differentiation Enhancing Factor 2 (DDEF2, or PAP or PAG3) is a multidomain protein, that is located in the Golgi apparatus and in the plasma membrane (Andreev J., et al., 1999, Mol. Cel. Biol., 19: 2338-2350). DDEF2 co-localizes with the PYK2 tyrosine protein kinase in the plasma membrane; PYK2 activiation results in phosphorylation of DDEF2 *in vivo* (Andreev J., et al., 1999., Mol. Cel. Biol., 19:2338-2350). It has also been shown, in experiments carried out *in vitro,* that DDEF2 is a potent activator of the small GTPases ARF1 and ARF5 and, to a lesser extent, ARF6 (Andreev J., et al., 1999., Mol. Cell. Biol., 19:2338-2350); these ARF proteins are involved in the regulation of transport processes between membrane-bound cell compartments; this transport is necessary for those cellular processes, such as secretion, endocytosis and shape changes, that accompany cell growth, division and migration (reviewed in Rothman J.E., 1994, Nature, 372:55-63; Rothman J.E., 1996, Protein Sci., 5:185-194). The mRNA transcribed from ddef2 gene, which codes for DDEF2 protein, is expressed at high levels in brain, kidney and heart tissue, and at low levels in lung, placenta and pancreas (Andreev J., et al., 1999, Mol. Cel. Biol., 19:2338-2350). It has also been reported that, in experiments carried out with DNA-chips, ddef2 was one of the 123 genes whose expresion was increased in pancreas biopsies from patients with cancer of the pancreas, when compared with healthy pancreas samples (Iacobuzio-Donahue C.A., et al., 2003, Am. J. Pathol., 162(4):1151-62). However those results were not confirmed by the authors with other methods with higher sensitivity, spcificity and reliability than DNA-chips, such as real time quantitative RT-PCR; it was also not shown whether the observed differential gene expression was associated with differential expression at the protein level.

Unexpectedly, the authors of the present invention have discovered, after thorough research and using different techniques (DNA-chips and quantitative RT-PCR), that the expression level of the ddef2 gene, is elevated in pancreatic carcinomas when compared with expression in non-tumorous pancreatic tissue from the same individuals, or in the pancreatic tissue of individuals with pancreatitis. The authors of the present invention have also discovered, using western-blot techniques, that the concentration of the DDEF2 protein is very high in biopsies of pancreatic ductal adenocarcinomas, compared with expression in non-tumoral pancreatic tissue from the same individuals, healthy individuals or individuals with pancreatitis. This makes DDEF2 a useful target for the development of new *in vitro* methods for diagnosis and prognosis and for the identification and development of therapeutic compounds for pancreatic ductal adenocarcinomas.

The detection *in vitro* of high levels of ddef2 mRNA as well as high levels of the corresponding DDEF2 protein, in samples of pancreatic tissue or in other samples, preferably serum samples, of individuals permits early detection of pancreatic cancer.

The development of new drugs targeted specifically against the ddef2 gene, or the DDEF2 protein, or any combination of these, is a new approach to treat pancreatic carcinomas.

The present invention, therefore, provides a highly sensitive *in vitro* method to detect the presence of a cancer of the pancreas, especially a pancreatic ductal carcinoma, to determine the stage or severity of this cancer in an individual or to monitor the effect of therapy administered to an individual with the said cancer, based on the detection and/or quantification of the DDEF2 protein, the ddef2 mRNA or the corresponding ddef2 cDNA, or any combination of these, in a sample of an individual. Also, the present invention provides targets or tools for the screening, identification, development and evaluation of the efficacy of therapeutic compounds for the treatment of cancer of the pancreas, especially for ductal adenocarcinoma of the pancreas. Finally, the invention provides agents characterised by the fact that they inhibit expression and/or activity of the DDEF2 protein for the treatment of cancer of the pancreas, especially for ductal adenocarcinoma of the pancreas.

## SUMMARY OF THE INVENTION

The object of the present invention is to develop an *in vitro* method to detect the presence of cancer of the pancreas, especially of ductal adenocarcinoma of the pancreas, to determine the stage or severity of this cancer in the individual or to monitor the effect of the therapy administered to an individual with this cancer.

A second object of the present invention is an *in vitro* method to screen for, identify, develop and evaluate the efficacy of compounds to treat cancer of the pancreas, especially pancreatic ductal adenocarcinomas.

An additional object of the invention lies in the use of sequences derived from the ddef2 gene to establish the diagnosis and prognosis *in vitro* of cancer of the pancreas, especially pancreatic ductal adenocarcinomas and to screen for, identify, develop and evaluate the efficacy of compounds for the treatment of this cancer.

Another object of the present invention is to provide agents characterised because they inhibit expression and/or activity of the protein DDEF2, to treat cancer of the pancreas, especially pancreatic ductal adenocarcinomas.

Finally, another object of this invention is a pharmaceutical composition consisting of one or several therapeutic agents together with an acceptable pharmaceutical excipient to treat cancer of the pancreas, especially pancreatic ductal adenocarcinomas.

## DESCRIPTION OF THE DRAWINGS

**Figure 1:** Denaturation curve of the PCR products amplified from the target ddef2 gene (Tm = 79°C) and of the reference gene, ribl10 (Tm=84°C), in experiments to measure gene expression by real time quantitative RT-PCR in pancreas samples.

**Figure 2:** Calculation of the amplification efficiency of the PCR reactions of ddef2, in experiments to measure gene expression by real time quantitative RT-PCR in pancreas samples.

**Figure 3:** Calculation of the amplification efficiency of the PCR reactions of ribl10, in experiments to measure gene expression by real time quantitative RT-PCR in pancreas samples.

**Figure 4:** Results of the analysis of expression of DDEF2 protein in human pancreas samples by westernblotting. Two biopsies of non-tumorous pancreatic tissue, from individuals without cancer of the pancreas (sample numbers 15 and 42), 4 biopsies of non-tumorous pancreatic tissue from individuals with cancer of the pancreas (sample numbers 34, 35, 36 and 37), 3 biopsies of non-tumorous, but inflamed, pancreatic tissue, from individuals with cancer of the pancreas and chronic pancreatitis (sample numbers 28, 29 and 30) and 3 biopsies of tumorous pancreatic tissue, from individuals with cancer of the pancreas stage I (sample numbers 27 and 33) and stage IV (sample number 23), were analysed. The expression of DDEF2 protein was also analysed in immortalised ductal pancreatic cell lines Capan-1, BxPC-3 and PANC-1; one biopsy of tumorous lung tissue, from an individual with metastasic cancer of the pancreas (sample number 38) and one biopsy of non-tumorous lung tissue, from the same individual were also analysed. A total of 40 µg of protein extract was assayed in each case. Membranes were developed with anti-DDEF2 antibody. Films were exposed for 1 min and DDEF2 appeared as an immunoreactive band with an apparent molecular weight of 110 kDa.

## DETAILED DESCRIPTION OF THE INVENTION

To facilitate the comprehension of the present patent application we give the meanings of some terms and expressions in the context of the invention:

The terms "subject" or "individual" refers to all animals classified as mammals and includes, but is not restricted to, domestic and farm animals, primates and humans. The subject is preferably a male or female human of any age or race.

The term "cancer" refers to the disease that is typically characterised by abnormal or unregulated cell growth, capable of invading adjacent tissues and spreading to distant organs.
The term "carcinoma" refers to the tissue resulting from abnormal or unregulated cell growth,
**[0019]** The term "cancer of the pancreas" or "cancer in the pancreas" or "adenocarcinoma of the pancreas" refers to any malign proliferative disorder of pancreatic cells.
The term "tumour" refers to any abnormal mass of tissue generated by a neoplastic process, whether this is benign (non cancerous) or malignant (cancerous).
The term "gene" refers to a region of a molecular chain of deoxyribonucleotides that encodes a protein and may represent a portion of a coding sequence or a complete coding sequence.
The term "DNA" refers to deoxyribonucleic acid. A DNA sequence is a sequence of deoxyribonucleotides.
The term "RNA" refers to ribonucleic acid. An RNA sequence is a sequence of ribonucleotides.
The term "mRNA" refers to messenger ribonucleic acid, which is the fraction of total RNA, which translates to proteins.
The term "cDNA" refers to a nucleotide sequence complementary to a sequence of mRNA.
The term "mRNA transcript of" refers to the RNA product transcribed from the corresponding gene (DNA) into mRNA, as a first step in the expression and translation to protein.
The term "nucleotide sequence" or "nucleotidic sequence" refers either to a sequence of ribonucleotides (RNA) or a sequence of deoxyribonucleotides (DNA).
The term "protein" indicates at least one molecular chain of amino acids linked through either covalent or non-covalent bonds. The term includes all forms of post-translational protein modifications, for example glycosylation, phosphorylation or acetylation.
The terms "peptide" and "polypeptide" refer to molecular chains of amino acids that represent a protein fragment. The terms "protein" and "peptide" are used indistinguishably.
The term "antibody" refers to a glycoprotein that exhibits a specific binding activity for a target molecule called an "antigen". The term "antibody" refers to monoclonal or polyclonal antibodies, either intact or fragments derived from them; and includes human antibodies, humanised antibodies and antibodies of non-human origin. The "monoclonal antibodies" are homogeneous, highly specific antibody populations directed against a single antigenic site or "determinant" of the target molecule. "Polyclonal antibodies" include heterogeneous antibody populations that are directed against different antigenic determinants of the target molecule.
The term "epitope", as it is used in the present invention, refers to an antigenic determinant of a protein, which is the sequence of amino acids of the protein that a specific antibody recognises. Such epitopes may be comprised of a contiguous stretch of amino acids (linear epitope) or of non-contiguous amino acids that are brought into proximity with one another by virtue of the three dimensional folding of the polypeptide chain (discontinuous epitopes).
The term "solid phase", as it is used in the present invention refers to a nonaqueous matrix to which the antibody can bind. Examples of materials for the solid phase include but are not limiterd to glass, polysaccharides (for example agarose), polyacrylamide, polystyrene, polyvinylic alcohol and silicons. Examples of solid phase forms are the well of a plate or a purification column.
The terms "oligonucleotide primer" and "primer" are used interchangeably in the present invention, and are used to refer to nucleotide sequences, that are complementary to target nucleotide sequences of the ddef2 or ribl10 genes. Each primer hybridises with its target nucleotide sequence and acts as an initiation site for nucleotide polymerisation catalysed by DNA polymerase, RNA polymerase or reverse transcriptase.
The term "probe", as it is used in the present invention, refers to a nucleotide sequence complementary to a nucleotide sequence derived from the ddef2 gene that can be used to detect the corresponding nucleotide sequence derived from the ddef2 gene.
The term "therapeutic target" refers to nucleotide or peptide sequences against which a drug or therapeutic compound can be designed and clinically applied.
The term "antagonist" refers to any molecule that inhibits the biological activity of the antagonised molecule. Examples of antagonistic molecules include, among others, proteins, peptides, variations of natural peptide sequences and small organic molecules (with a molecular weight usually, but not limited to, lower than 500 Daltons).
The present invention is based on the discovery that both gene expression of ddef2, and the concentration of the DDEF2 protein are increased in ductal adenocarcinoma of the pancreas of an individual.
Therefore, the present invention first of all provides an *in vitro* method to detect the presence of a cancer of the pancreas, especially of a ductal adenocarcinoma of the pancreas, in an individual, to determine the stage or severity of this cancer in the individual, or to monitor the effect of the therapy administered to an individual with this cancer, that comprises:

a) detection and/or quantification either of DDEF2 protein, of the ddef2 mRNA or the corresponding cDNA, in a sample of an individual and;

b) comparison of either the amount of DDEF2 protein, of the mRNA of the ddef2 gene or of the corresponding cDNA, detected in a sample of an individual, with the respective amount either of the DDEF2 protein, of the mRNA of the ddef2 gene or of the corresponding cDNA, detected in the samples of control individuals or in previous samples of the same individual or with normal reference values.

The method provided by the present invention is highly sensitive and specific and is based on the fact that subjects or individuals diagnosed with cancer of the pancreas, especially with ductal adenocarcinoma of the pancreas, present high levels of mRNA transcribed from the ddef2 gene (elevated levels of expression of the ddef2 gene) or elevated levels of the protein coded by the ddef2 gene (protein *DDEF2),* in comparison with the corresponding levels in samples from subjects without a clinical history of this cancer.

The present method comprises a step in which a sample is obtained from the individual. Different liquid samples can be used such as: urine, blood, plasma, serum, pleural fluid, ascitic fluid, synovial fluid, bile, semen, gastric exudate or cerebrospinal fluid. The sample can also consist of tissue of the affected organ, such as the pancreas, that can be obtained by any conventional method, preferably surgical resection.

Samples can be obtained from subjects previously diagnosed or not diagnosed with a specific type of cancer; or also from a subject receiving treatment, or who has previously received treatment for a cancer, especially for cancer of the pancreas.

The present method also comprises a step for extraction of the sample, either to obtain the extract of proteins from it or to obtain the extract of total RNA. One of these two extracts provides the working material for the next phase. The extraction protocols for total protein or total RNA are well known by experts in the area (Chomczynski P. et al., Anal. Biochem., 1987, 162: 156; Chomczynski P., Biotechniques, 1993, 15: 532).

Any conventional assay can be used in the context of the invention to detect a cancer, provided that it measures *in vitro* the levels of mRNA transcribed from the ddef2 gene or its complementary cDNA, the concentration of the protein DDEF2, in samples collected from individuals to be studied and control individuals.

Therefore, this invention provides a method to detect the presence of a cancer of the pancreas in an individual, especially with ductal adenocarcinoma of the pancreas, to determine the stage or severity of this cancer in an individual, or to monitor the effect of the therapy administered to an individual who presents this cancer, based either on measuring the levels of the DDEF2 protein or on measuring the level of expression of the ddef2 gene.

If the aim is to detect the DDEF2 protein, the method of the invention comprises a first step in which the protein extract of the sample is placed in contact with a composition of one or more specific antibodies against one or more epitopes of the DDEF2 protein and a second stage to quantify the complexes formed by antibodies and the DDEF2 protein.

There is a wide range of immunological assays available to detect and quantify formation of specific antigen-antibody complexes; numerous competitive or non-competitive protein-binding assays have been described previously and a large number of these are available commercially.

Hence, the DDEF2 protein can be quantified with antibodies such as, for example: monoclonal antibodies, polyclonal antibodies, either intact or recombinant fragments of these, combibodies and Fab fragments or scFv of antibodies, specific against the DDEF2 protein; these antibodies are human, humanised or of animal origin. The antibodies used in these assays can be labelled or unlabelled; the unlabelled antibodies can be used in agglutination assays; the labelled antibodies can be used in a wide range of assays. Marker molecules that can be used to label antibodies include radionucleotides, enzymes, fluofluorides, chemoluminescent reagents, enzymatic substrates or cofactors, enzymatic inhibitors, particles, colorants and derivatives.

There are a wide variety of assays well known to those skilled in the art that can be used in the present invention, which use unlabelled antibodies (primary antibody) and labelled antibodies (secondary antibodies); these techniques include but are not limited to the western-blot or western transfer, ELISA (Enzyme-Linked immunosorbent assay), RIA (Radioimmunoassay), Competitive EIA (Competitive enzyme immunoassay), DAS-ELISA (Double antibody sandwich-ELISA), immunocytochemical and immunohistochemical techniques, techniques based on the use of biochips or protein microarrays that include specific antibodies or colloidal precipitation in formats such as *dipsticks.* Other ways to detect and quantify the DDEF2 protein include affinity chromatography techniques, ligand binding assays or lectin binding assays.

The preferred immunoassay for the invention is a double antibody sandwich ELISA (*DAS-ELISA*). In this immunoassay any antibody or combination of antibodies can be used, specific against one or more epitopes of the DDEF2 protein. As an example of one of the many possible formats of this assay, a monoclonal or polyclonal antibody, or a fragment of this antibody, or a combination of these antibodies that recognise one or more epitopes of the DDEF2 proteinare attached to the surface of a solid phase and placed in contact with the sample to be analysed and incubated for a specific time and in appropriate conditions to form the antigen-antibody complexes. After washing in appropriate conditions to eliminate nonspecific complexes, an indicator reagent, consisting in a monoclonal or polyclonal antibody, or a fragment of this antibody, or a combination of these and which recognises one or more epitopes of the target DDEF2 protein, bound to a signal generator compound, is incubated with the antigen-antibody complexes in appropriate conditions of time and temperature. The presence of the DDEF2 protein in the sample to be analysed is detected and, if present, quantified and the signal generated is measured. The amount of DDEF2 protein present in the sample to be analysed is proportional to this signal.

When the aim is to detect mRNA or the cDNA corresponding to the ddef2 gene and not the proteins that code this, the method of the invention to detect the carcinoma *in vitro* has several different steps. Hence, after obtaining the sample and extracting the total RNA, the method of the invention effects the detection of the mRNA or of the corresponding cDNA of the ddef2 gene, comprising a first step of amplification of the extract of total RNA or the corresponding cDNA synthesised by reverse transcription from the mRNA and a second step of quantification of the amplification product of mRNA or of the cDNA of the ddef2 gene..

One example of mRNA amplification consists in reverse transcription (RT) of the mRNA into cDNA, followed by Polymerase Chain Reaction (PCR), using oligonucleotide primers, using the primer sequences 5'-GCATGCCATTCTCCATCAGA-3' y 5'- CCACCCCTTAACAGATCACACA -3'. PCR is a technique for the amplification of a specific nucleotide sequence (target) contained in a mixture of nucleotide sequences. In PCR, an excess of a pair of oligonucleotide primers is used that hybridise with complementary strands of the target nucleotide sequence. After this, an enzyme with polymerase activity (DNA Polymerase) extends each primer, using the target nucleotide sequence as a template. The extension products are, therefore, converted into target sequences, after dissociation of the original strand. New primer molecules hybridise and the polymerase extends them. The cycle is repeated to exponentially increase the number of target sequences. This technique is described in the patents US 4683195 and US 4683202. Many methods have been described previously to detect and quantify amplification products by PCR and any of these can be used in the invention. In a preferred method of the invention, the amplified product is detected by agarose gel electrophoresis as follows: five microliters of amplification product are separated by agarose gel electrophoresis at a concentration of 2% agarose, in a Tris-Borate-EDTA (TBE) buffer at 100 volts direct current for one hour. After electrophoresis the gel is stained with ethidium bromide and the amplification product is observed when the gel is illuminated with ultraviolet (uv) light. As an alternative to staining, a preferred method is to transfer the amplified product to a nylon membrane by *Southern blotting or Southern transfer* techniques to be detected with a specific cDNA probe of the ddef2 gene, appropriately labelled.

In another example, mRNA detection is performed following electrophoretic separation of mRNA by transferring the mRNA to a nylon membrane using transfer techniques such as northern-blot or northern transfer and detecting it with specific RNA probes or of the corresponding cDNA of the ddef2 gene.

In one specific application, amplification and quantification of the mRNA corresponding to the ddef2 gene, is carried out by quantitative RT-PCR in real time (Q-PCR).

The final step of the method of the invention to detect *in vitro* the presence of the cancer in a sample from an individual comprises comparing the amount of protein DDEF2, the amount of mRNA of the ddef2 gene or the amount of the corresponding cDNA, detected in a sample of an individual, with the amount of protein DDEF2, the amount of mRNA of the ddef2 gene, the amount of corresponding cDNA, detected in the samples of control subjects or in previous non-tumorous samples of the same individual or with normal reference values.

For its second object, the invention also provides a method *in vitro* to identify and evaluate the efficacy of therapeutic agents against cancer of the pancreas, especially against a ductal adenocarcinoma of the pancreas, that comprises:

a) placing into contact a culture of tumour cells, with the candidate compound, in the appropriate conditions and for the time required for these to interact,
b) detection and quantification of the expression levels of the ddef2 gene or the DDEF2 protein or both, and
c) comparing these expression levels with those of the control culture of tumour cells not treated with the candidate compound.

Quantification of the expression levels of the ddef2 gene or the DDEF2 protein, is performed in a similar manner to that described in the method of the invention to detect *in vitro* the presence of a cancer of the pancreas, especially of a ductal adenocarcinoma of the pancreas, in an individual.

When an agent reduces the expression levels of the ddef2 gene or reverses the effects of high expression of this gene, preferably reducing the levels of cellular proliferation, this agent becomes a candidate for cancer therapy.

Therefore, another object of this invention refers to the use of nucleotide or peptide sequences derived from the ddef2 gene, in methods to screen for, identify, develop and evaluate the efficacy of therapeutic compounds against cancer of the pancreas, especially against ductal adenocarcinoma of the pancreas. It is noteworthy, the recent importance given to screening methods based on the competitive or non-competitive binding of the potential therapeutic molecule to the therapeutic target.

Another object of this invention refers to the use of nucleotide or peptide sequences derived from the ddef2 gene to detect the presence of a cancer of the pancreas, especially of a ductal adenocarcinoma of the pancreas, to determine the stage or severity of this cancer in the individual or to monitor the effect of the therapy administered to an individual with this cancer.

Another object of this invention consists in providing agents characterised because they inhibit expression and/or activity of the DDEF2 protein. These agents, which can be identified and evaluated according to the present invention, can be selected from the group comprised by:

a) an antibody, or combination of antibodies, specific against one or more epitopes present in the DDEF2 protein, preferably a human or humanised monoclonal antibody. These can also be a fragment of antibody, a single chain antibody or an anti-idiotype antibody,
b) cytotoxic agents, such as toxins, molecules with radioactive atoms or chemotherapeutic agents, including, but not limited to, small organic and inorganic molecules, peptides, phospopeptides, antisense molecules, ribozymes, siRNAs, triple helix molecules etc. that inhibit expression and/or activity of the DDEF2 protein, and
c) compounds that are antagonists of the DDEF2 protein, that inhibit one or more of the functions of the DDEF2 protein.

Finally, another object of the present invention is a pharmaceutical composition that includes a therapeutically effective amount of one or several of the previously mentioned agents together with one or more excipients and/or transporter substances. Also, this composition can contain any other active ingredient that inhibits the function of the DDEF2 protein.

The excipients, transporter compounds and auxiliary substances must be pharmaceutically and pharmacologically tolerable so that they can be combined with other components of the formulation or preparation and not have any adverse effects on the organism treated. The pharmaceutical compositions or formulations include those that are suitable for oral or parenteral administration (including subcutaneous, intradermic, intramuscular or intravenous), although the best route of administration depends on the patient's condition. Formulations can also be in the form of single doses. Formulations are prepared according to well known pharmacological methods. Amounts of active substances to be administered vary depending on the characteristics of the therapy.

The following examples serve to illustrate the invention.

### Example 1.- Differential analysis of the expression of the ddef2 gene in samples of pancreas tissue, using *Human* Genome *U133 DNA microarrays*

#### 1.1. Material and methods

**Microarrays.** GeneChip Test 3 (Affymetrix, Santa Clara) microarrays were used, that permitted the quality of RNA to be tested before analysing expression with the GeneChip Human Genome U133A array (Affymetrix, Santa Clara), which represents 13,220 recorded complete gene sequences; the ddef2 gene is represented in the microarray by the set of probes **206414_s_at** of Affymetrix, which are sense oligonucleotides 25 nucleotides long, designed on the basis of the Hs.12802 sequence of Unigene, or **N. Acc. AB007860** of GeneBank (Table 1).

**Table 1. Description of the probes corresponding to the set of probes 206414_s_at.**

| Consecutive order of probes | Zone of the interrogated reference sequence | Probe sequence (5'-3') |
|---|---|---|
| 1 | 5150 | CTCCAGTCCCAAACTAATCTGTCAG |
| 2 | 5165 | AATCTGTCAGGTTCACTGGTACATA |
| 3 | 5265 | GAAATTATATTAACGACCCTGCTAA |
| 4 | 5279 | GACCCTGCTAATATCCTTTCTTAGT |
| 5 | 5294 | CTTTCTTAGTTATTTGCTCCTTGCA |
| 6 | 5395 | CAGCTGCAGGATTCTGGCATTTTGC |
| 7 | 5410 | GGCATTTTGCATGCCATTCTCCATC |
| 8 | 5446 | GATGGCTCAGAACATGTACACAGAC |
| 9 | 5544 | GATATTCAGTTCATTCACCTGATTA |
| 10 | 5559 | CACCTGATTACTTTGGTTGCAGCAC |
| 11 | 5667 | TTACTCTTCTTGTTAACTAGTCATT |

**Samples.** The samples studied were from clinically classified biopsies, obtained by surgical resection: biopsies of non-tumorous pancreatic tissue from individuals with cancer of the pancreas ("non-tumorous") (n = 1); biopsies of non-tumorous pancreatic tissue, but with pancreatitis, from individuals without cancer of the pancreas, but with chronic pancreatitis ("chronic pancreatitis") (n = 1); biopsies of non-tumorous pancreatic tissue, but with pancreatitis, from patients with cancer of the pancreas and pancreatitis ("pancreatitis") (n = 1); biopsies of tumorous pancreatic tissue, from patients with cancer of the pancreas ("tumour") (n = 10) in one of the following stages: Stage I, tumour limited to pancreas. Stage III, tumour spread to regional lymph nodes and Stage IVB, metastases in distant organs and tissues. All samples were clinically and histologically classified (grade and stage) in the Hospital Central de Asturias, the same hospital where they had been collected according to the precepts of the Helsinki declaration. Samples were frozen in liquid nitrogen immediately after extraction and stored at -80°C until analysis.

For each stage of tumour the following samples were analysed:

- Stage I tumour: 7 samples
- Stage III tumour: 1 sample
- Stage IV tumour: 2 samples

***GeneChip* gene expression analysis**

Analysis was done with total RNA from individual subjects. The 13 samples analysed are described in Table 2.

Table 2.

| Description of the samples analysed | | | |
|---|---|---|---|
| **Sample Code** | **Type** | **TNM Classification** | **Stage** |
| PA36 | Non-tumorous | - | - |
| PA29 | Pancreatitis | - | - |
| PA46 | Chronic Pancreatitis | - | - |
| PA02 | Tumour | T2N0M0 | I |
| PA03 | Tumour | T2N0M0 | I |
| PA17 | Tumour | T1bN0M0 | I |

Table 2. (continued)

| Description of the samples analysed | | | |
|---|---|---|---|
| **Sample Code** | **Type** | **TNM Classification** | **Stage** |
| PA19 | Tumour | T1bN0M0 | I |
| PA25 | Tumour | T1N0M0 | I |
| PA27 | Tumour | T1N0M0 | I |
| PA33 | Tumour | T1bN0M0 | I |
| PA24 | Tumour | T3N1M0 | III |
| PA16 | Tumour | T2N1M1 | IV |
| PA23 | Tumour | T1bN1M1 | IV |

*CRNA synthesis*

Total RNA of each biopsy was obtained by homogenising the tissue in TRIzol® Reagent (Life Technologies), following the supplier's recommendations. The resulting total RNA was cleaned with the Rneasy kit (QIAGEN) (Chomczynski P. et al., Anal. Biochem., 1987, 162: 156; Chomczynski P., Biotechniques, 1993, 15: 532). Of each preparation of total RNA, 10 µg were used as starting material for synthesis of the first cDNA strand with the reverse transcriptase enzyme SuperScript™ II RNase (Life Technologies), using as a primer an oligonucleotide oligo-dT containing the sequence of the RNA polymerase promoter of phage T7. The second cDNA strand was synthesised using the enzymes DNA polymerase I of *E. coli* (Invitrogen Life Technologies), DNA ligase of *E. coli* (Invitrogen Life Technologies), Rnase H of *E. coli* (Invitrogen Life Technologies), and DNA polymerase of phage T4 (Invitrogen Life Technologies). The biotin labelled cRNA was synthesised using the ENZO BioArray™ HighYield™ Transcript Labeling Kit (Enzo Diagnostics Inc). After *in vitro* transcription, the nucleotides not incorporated were eliminated using the RNeasy columns (QIAGEN).

*Array Hybridization and scanning*

A total of 15 µg of each biotinylated cRNA were fragmented at 94°C for 35 minutes in a buffer solution containing 40 mM Tris-Acetate (pH 8.1), 100 mM potassium acetateand 30 mM magnesium acetate. The fragmented cRNA was mixed with hybridization buffer (100 mM MES, 1M NaCl, 20 mM EDTA, 0.01% Tween 20) and heated to 99° for 5 minutes and then to 45° for 5 minutes, after which it was loaded in the Affymetrix array. The first array in which the hybridization was carried out was Test 3 of Affymetrix. With this array the quality of RNA can be tested before analysing expression in the Affymetrix® GeneChip® *Human Genome* 133 A (HG-U133A).

For hybridization, arrays were incubated in a rotary incubator at 45° for 16 hours with a constant rotation of 60 rpm.

Washing and staining of each array was done in the Affymetrix® fluid station. A washing and staining programme was used that included:

- 10 x 2 washing cycles with SSPE-T 6x (0.9 m NaCl, 60 mM $NaH_2PO4$, 6 mM EDTA, 0.01% Tween 20) at 25°C,
- 4x15 cycles with 0.1 mM MES, 0.1 M NaCl, 0.01 % Tween 20 at 50°C,
- Staining with biotinylated cRNA with a phycoerythrin streptavidin conjugate (10 µg/ml Molecular Probes)
- 10 x 4 washing cycles with SSPE-T at 25C°
- Staining an anti-streptavidin conjugate for 10 minutes
- Staining a phycoerythrin-streptavidin conjugate (1 mg/ml, Molecular Probes) for 10 minutes
- 15 x 4 washing cycles with SSPE-T at 30C°

Arrays were scanned at 560 nm using a confocal microscope that uses laser emission (Agilent GeneArray Scanner). Analysis of intensity readings was done with the Microarray Suite 5.0 software. For comparison of arrays these were scaled to a total intensity of 100.

**1.2. Results**

Differential analysis of the expression of the ddef2 gene in biopsies of tumorous pancreatic tissue, compared to biopsies of non-tumorous pancreatic tissue with or without pancreatitis, was done using the array comparison data obtained using the Affymetrix software. The parameters studied (in the order in which these appear in the list) were: i) Detection. This indicates whether the transcript is Present (P), Absent (A) or Marginal (M), ii) Change: This indicates whether expression of a specific transcript Increases (I), Decreases (D), Does not change (NC), Increases marginally (IM), or decreases marginally (DM), iii) Signal Log Ratio (SLR): This indicates the level of change in expression between the baseline (control) and the sample under test. This change is expressed as the $log_2$ of the ratio *(fold change* or number of times that this gene expression is elevated or repressed in the problem-tumour sample versus a control-non-tumorous sample). An SLR value of 1 is considered to be significant (equivalent to a *fold change* of 2), for transcripts in which expression increases compared to control and of -1, for transcripts for which expression decreases compared to control.

**Table 3. Results obtained for the ddef2 gene. N. Acc. AB007860. Result of comparisons with PA36 non-tumorous control sample**

| Sample | Detection | SLR | Change |
|---|---|---|---|
| Non-tumorous controls | | | |
| **PA36** | P | - | - |
| **PA29** | P | 0.7 | NC |
| **PA46** | P | -0.5 | NC |
| Stage I tumours | | | |
| **PA02** | P | 0.8 | I |
| **PA03** | P | 2.9 | I |
| **PA17** | P | 0.8 | NC |
| **PA19** | P | 1.1 | I |
| **PA25** | P | 0.8 | I |
| **PA27** | P | 1.4 | I |
| **PA33** | P | 0.8 | I |
| Stage III tumours | | | |
| **PA24** | P | 1.6 | I |
| Stage IV tumours | | | |
| **PA23** | P | 1.9 | I |
| **PA16** | P | 3.1 | I |

**Table 4. Results obtained for the ddef2 gene. N. Acc. AB007860. Results of comparisons with PA46 chronic pancreatitis control sample**

| Sample | Detection | SLR | Change |
|---|---|---|---|
| Non-tumorous controls | | | |
| PA46 | P | - | - |
| PA29 | P | 1.5 | I |
| PA36 | P | 0.5 | NC |
| Stage I tumours | | | |
| PA02 | P | 1.4 | I |
| PA03 | P | 3.7 | I |
| PA17 | P | 1.5 | I |
| PA19 | P | 1.6 | I |
| PA25 | P | 1.4 | I |
| PA27 | P | 2.5 | I |
| PA33 | P | 1.5 | I |
| Stage III tumours | | | |
| PA24 | P | 2.2 | I |
| Stage IV tumours | | | |
| PA23 | P | 2.4 | I |
| PA16 | P | 3.6 | I |

Differential analysis of the expression of the ddef2 gene in different stages of pancreatic tumour development compared with the non-tumorous control sample (PA36), showed that the expression levels of the ddef2 gene were increased in 9 of the 10 biopsies of the pancreatic tumours that were analysed, with SLR values ranging between 0.8 and 3.1 (fold changes between 1.5 and 8) (Table 3). The differential expression analysis of the ddef2 gene compared to the control sample with chronic pancreatitis (PA46), showed that the expression levels of the ddef2 gene were increased in the 10 biopsies of pancreatic tumours that were analysed, with SLR values ranging between 1.4 and 3.6 (fold changes between 3 and 12) (Table 4).

**1.3. Discussion**

These results showed that the expression level of the ddef2 gene was increased in most of the pancreatic ductal adenocarcinomas that were analysed, when compared with the non-tumorous control biopsy sample or with the chronic pancreatitis control biopsy sample. In addition levels of ddef2 gene expression showed some correlation between the stage of the disease and the level of gene expression, with the higher expression levels generally associated with more advanced cancer stages (III and IV).

**Example 2.- Differential analysis of expression of the ddef2 gene in samples of pancreas tissue using Real time - quantitative RT-PCR**

**2.1. Material and methods**

The method used consists of the reverse transcription of mRNA to cDNA followed by its amplification in a *LightCycler (Roche)* thermal cycler using SYBR Green to detect the amplified product. Quantification was done in real time and permits the relative expression of the sequence to be calculated in different samples in the linear amplification phase of the reaction.

**Samples**. The samples studied were from clinically staged biopsies, obtained by surgical resection: biopsies of non-tumorous pancreatic tissue from individuals with ductal adenocarcinoma of the pancreas ("non-tumorous") (n = 1); biopsies of non-tumorous pancreatic tissue, but with pancreatitis, from individuals without ductal adenocarcinoma of the pancreas, but with chronic pancreatitis (n = 1) ("chronic pancreatitis"); biopsies of patients with ductal adenocarcinoma of the pancreas (n = 5) in one of the following stages: Stage I, tumour limited to the pancreas and Stage IV, metastases in distant tissues or organs. All the samples were classified histologically (grade and stage) in the Hospital Central de Asturias, the same hospital in which the samples had been collected according to the precepts of the Helsinki declaration. Samples were frozen in liquid nitrogen immediately after extraction and kept at -80°C until analysis.

**Real Time Quantitative RT-PCR.** The analysis was carried out with total RNA from individual subjects. The 7 samples analysed are described in Table 5.

Table 5.

| **Description of the samples analysed**. | | | |
|---|---|---|---|
| **Code of sample** | **Type** | **TNM Classification** | **Stage** |
| PA36 | Non-tumorous | - | - |
| PA46 | Chronic Pancreatitis | - | - |
| PA19 | Tumour | T1bN0M0 | I |
| PA03 | Tumour | T2N0M0 | I |
| PA33 | Tumour | T1bN0M0 | I |
| PA14 | Tumour | T3N1M1 | IV |
| PA23 | Tumour | T1bN1M1 | IV |

*CDNA synthesis*

The total RNA of each of the biopsies was obtained by homogenising the tissue in TRlzol( Reagent (Life Technologies), following the supplier's recommendations. The resulting total RNA was cleaned with the RNeasy kit (QIAGEN) (Chomczynski P. et al., Anal. Biochem., 1987, 162: 156; Chomczynski P., Biotechniques, 1993, 15: 532). The RNA was quantified spectrophotometrically and 5 µg of total RNA were digested with DNaseI. A total of 1 µg of RNA treated with DNAse was used as starter material for the synthesis of the first strand of cDNA with the inverse transcriptase enzyme SuperScript( II RNase (Life Technologies), using an oligo-dT oligonucleotide that contained the sequence of the RNA polymerase promoter of the phage T7 as a primer. Aliquots of cDNA diluted to the working concentration were prepared.

Amplification

The cDNA synthesised was amplified using specific primers of the human ddef2 gene (5'-GCATGCCATTCTCCATCAGA-3' y 5'-CCACCCCTTAACAGATCACACA -3') and of the gene that codes for the human ribosomal protein L10 (5'-TGCGATGGCTGCACACA-3' and 5'-TCCCTTAGAGCAACCCATACAAC-3'). PCR reactions in real time were prepared using the LightCycler-FastStart DNA master SYBR Green I kit (Roche) following the manufacturer's instructions. The amplification programme consisted in 1 cycle of 95°C (denaturation) for 10 s, 60°C (annealing) for 5 s, 72°C (amplification and acquisition of fluorescence) for 10 s. The programme of analysis of the denaturation curves consists of a cycle of one pulse of 95°C, 65°C for 15 s, and a pulse of 95°C for the amplification and acquisition step.

Quantification

Firstly, the specificity of the PCR products are determined by analysing the denaturation curves. Subsequently, as a relative measure of gene expression, the ratio of the abundance of mRNA transcripts of ddef2 to the abundance of ribl10 transcripts was calculated and the ratio was normalised for each of the tumoral samples on the basis of the values of the control sample. To calculate the efficiency of the PCR reactions (ddef2 and ribl10) a reference curve was developed for each gene sequence with serial dilutions of cDNA. The concentrations of template cDNA for reactions in the reference curve were given the arbitrary values 10, 5, 2.5, 1.25 and 0.625. The efficiency was calculated using

the following equation:

$$E = 10-1/p$$

where E is the efficiency of the amplification and p is the gradient of the reference line.

The ratio of the values of gene expression was determined using the following equation, which relates the experimental data of the amplification and corrects the error resulting from the difference in efficiency of the PCR reactions:

$$\text{Ratio} = \frac{E_{target}^{-(Cp\ target\ control\ -Cp\ target\ sample)}}{E_{reference}^{-(Cp\ reference\ control\ -\ Cp\ reference\ sample)}}$$

where *E* is the amplification efficiency, *Cp* is the *Crossing point*, *target* is ddef2, *reference* is ribl10, *control* is the control sample (non-tumorous or chronic pancreatitis) and *sample* is the tumour sample.

**2.2. Results**

**Analysis of the denaturation curves**

Analysis of the PCR products showed specific amplification of two products with denaturation temperatures similar to those expected according to the software used to design the primers, *PrimerExpress (Applied Biosystems*) (Figure 1).

**Calculation of the efficiency of the PCR reactions**

For each of the 5 dilutions of cDNA, two replicate samples were amplified and the cut off points (Cp) plotted on a graph against the logarithm of the concentration of cDNA to construct the reference line (Tables 6 and 7, figure 2).

Table 6.

| **Cut off points (Cp) of the amplification of 5 dilutions of cDNA for each replicate amplified with primers of the ddef2 gene** | | |
|---|---|---|
| **[RNA] (ng)** | **Log [RNA]** | **Cp** |
| 10 | 1 | 23.07 |
| 10 | 1 | 22.89 |
| 5 | 0.698970004 | 23.98 |
| 5 | 0.698970004 | 24.07 |
| 2.5 | 0.397940009 | 25.07 |
| 2.5 | 0.397940009 | 25 |
| 1.25 | 0.096910013 | 26.03 |
| 1.25 | 0.096910013 | n.d. |
| 0.625 | -0.204119983 | n.d. |
| 0.625 | -0.204119983 | n.d. |

Table 7.

| **Cut off points (Cp) for the amplifications of 5 dilutions of cDNA for each replica amplified with primers of the ribl10 gene** | | |
|---|---|---|
| **[RNA] (ng)** | **Log [RNA]** | **Cp** |
| 10 | 1 | 20.87 |

Table 7. (continued)

| Cut off points (Cp) for the amplifications of 5 dilutions of cDNA for each replica amplified with primers of the ribl10 gene | | |
|---|---|---|
| **[RNA] (ng)** | **Log [RNA]** | **Cp** |
| 10 | 1 | 20.98 |
| 5 | 0.698970004 | 22.65 |
| 5 | 0.698970004 | 22.98 |
| 2.5 | 0.397940009 | 24.33 |
| 2.5 | 0.397940009 | 24.74 |
| 1.25 | 0.096910013 | 25.29 |
| 1.25 | 0.096910013 | 25.44 |
| 0.625 | -0.204119983 | 27.02 |
| 0.625 | -0.204119983 | 27.38 |

**Quantification of the change in expression of the ddef2 gene**

Two replicates of each sample (controls and tumour biopsy samples) were amplified with the ddef2 specific primers . From the cut off points generated in these amplifications (Table 8), changes in expression of the ddef2 gene were calculated in tumour samples, compared to non-tumorous and chronic pancreatitis controls, applying the previously described equation (Table 9).

Table 8.

| **Experimental cut off points for ddef2** in 5 tumour and 2 control samples. | | | | | | | |
|---|---|---|---|---|---|---|---|
| **ribl10** | | | | | | | |
| **Replicas** | **PA36** | **PA46** | **PA03** | **PA14** | **PA19** | **PA23** | **PA33** |
| 1 | 30,90 | 28,46 | 29,49 | 26,67 | 31,19 | 28,95 | 28,70 |
| 2 | 29,69 | 28,50 | 29,61 | 26,97 | 31,71 | 29,24 | 28,53 |
| Mean | *30, 30* | *28,48* | *29,55* | *26,82* | *31,45* | *29,10* | *28,62* |
| *SD* | *0,86* | *0, 03* | *0, 08* | *0,21* | *0,37* | *0, 21* | *0,12* |
| **ddef2** | | | | | | | |
| **Replicas** | **PA36** | **PA46** | **PA03** | **PA14** | **PA19** | **PA23** | **PA33** |
| 1 | 30,61 | 29,41 | 26,94 | 25,69 | 29,49 | 27,70 | 28,47 |
| 2 | 30,84 | 28,89 | 26,87 | 25,82 | 29,44 | 27,47 | 28,56 |
| *Mean* | *30,73* | *29,15* | *26,91* | *25,76* | *29,47* | *27,59* | *28,52* |
| *SD* | *0,16* | *0,37* | *0, 05* | *0, 09* | *0, 04* | *0,16* | *0, 06* |

Table 9.

| Values of overexpression (Fold Change) of ddef2 in 5 tumor samples, compared with PA36 non-tumorous control sample and compared with PA46 chronic pancreatitis control sample | | | | | |
|---|---|---|---|---|---|
| **Samples** | **PA03** | **PA14** | **PA19** | **PA23** | **PA33** |
| Increased expression relative to PA36 **non-tumorous control sample** | 9,6 | 6,1 | 4 | 4,9 | 2,1 |
| **Increased expression relative to PA46 chronic pancreatitis control sample** | 7,5 | 4,8 | 3,1 | 3,8 | 1,6 |

The results confirmed the data obtained by measuring the difference in gene expression with DNA chips (example 1), that is, the expression of the ddef2 gene was significantly increased in the tumour tissue examined, compared to expression in a non-tumorous pancreas sample and compared to a pancreatitis sample; the average increase of ddef2 gene expression was 5-fold, when compared to non-tumorous samples without pancreatitis (PA36), and 4-fold, when compared with non-tumorous samples with chronic pancreatitis (PA46) (table 9).

**2.3. Discussion**

These results confirmed that the increased expression of ddef2 gene is associated to the ductal adenocarcinoma of the pancreas.

Example 3.- Differential analysis of expression of the DDEF2 protein in samples of pancreas tissue using the western blot technique with specific antibodies.

**3.1 Materials and methods.**

Samples: The samples studied were from clinically staged biopsies, obtained by surgical resection: biopsies of non-tumorous pancreatic tissue from individuals without ductal adenocarcinoma of the pancreas (n = 2, samples number 15 and 42); biopsies of non-tumorous pancreatic tissue from individuals with ductal adenocarcinoma of the pancreas (n = 4, samples 34, 35, 36 and 37); biopsies of non-tumorous, but inflamed, pancreatic tissue, from individuals with ductal adenocarcinoma of the pancreas and chronic pancreatitis (n = 3, samples 28, 29 and 30); biopsies of tumorous pancreatic tissue, from individuals with ductal adenocarcinoma of the pancreas stage I (n = 2, samples 27 and 33) and stage IV (n = 1, sample 23). DDEF2 expression in cell extracts from immortalised pancreatic ductal cell cultures Capan-1, BxPC-3 and PANC-1 was also analysed; one biopsy of tumorous lung tissue, from an individual with metastasic cancer of the pancreas (n = 2, sample 38) and one biopsy of non-tumorous lung tissue from the same individual were also analysed; samples were classified histologically (grade and stage) in the Hospital Central de Asturias, the same hospital from which the samples had been collected following the precepts of the Helsinki declaration. The samples were frozen in liquid nitrogen immediately after extraction and stored at -80°C until analysis.

**Protein extraction:** Frozen samples were homogenised in RIPA B buffer [sodium phosphate 20 mM (pH 7.4), NaCl 150 mM, Triton X-100 1%, EDTA 5 mM and a combination of protease inhibitors (Roche Diagnostics Inc., Mannheim, Germany)]. Samples were then centrifuged at 15,000 x g for 10 minutes at 4°C, to eliminate cellular debris. Then, the supernatant was recovered and the concentration of proteins was estimated by the Bradford dye binding assay (Bio-Rad, Hercules, CA, US).

**Western transfer assays:** Samples of protein extracts with 40 μg of total protein were taken, mixed with SDS-PAGE stacking buffer supplemented with 5% of β-mercaptoethanol and were incubated at 100°C for 5 min, after which they were loaded in an 8% polyacrylamide gel. The proteins were then transferred to nitrocellulose membranes and probed with specific antibodies against the DDEF2 protein. Finally, the membranes were hybridised with a secondary antibody conjugated with peroxidase (Amersham, Little Chalfont, UK) and the chemoluminescent signal was detected using the ECL system (Amersham) with high performance chemiluminescence film (Hyperfilm ECL, Amersham).

**3.2 Results**

**Expression of DDEF2 in human ductal adenocarcinomas of the pancreas:** The results obtained are compiled in Fig. 4 and in Table 10. As can be observed, expression levels of the DDEF2 protein were almost undetectable, or were undetectable, in biopsies of non-tumorous pancreatic tissue from individuals without ductal adenocarcinoma of the pancreas, biopsies of non-tumorous pancreatic tissue from individuals with ductal adenocarcinoma of the pancreas, biopsies of non-tumorous, but inflamed, pancreatic tissue, from individuals with ductal adenocarcinoma of the pancreas and chronic pancreatitis and in biopsies of ductal adenocarcinomas of the pancreas stage I. In contrast, DDEF2 was overexpressed in the stage IV ductal adenocarcinoma of the pancreas and in the immortalised ductal cell cultures Capan-1, BxPC-3 and PANC-1.

**3.3. Discussion**

The results confirmed, at the protein expression level, the data obtained by measuring the difference in gene expression, i.e. the expression of the DDEF2 protein was greatly increased in a tumour sample from an individual with late stage ductal adenocarcinoma of the pancreas, when compared to non-tumorous samples from individuals with or without pancreatitis. Nevertheless, the overexpression of DDEF2 protein in sample number 23 (stage IV), but not in samples 27 and 33 (stage I), suggests a correlation between DDEF2 protein expression level and tumour invasiveness.

Table 10.

| **Expression of DDEF2 in human pancreatic adenocarcinomas** | | | | |
|---|---|---|---|---|
| **Type of sample** | *N* | **DDEF2** absent | **DDEF2** present | **DDEF2 overexpressed** |
| Pancreas of healthy individuals (PA15, PA42) | 2 | 2 | 0 | 0 |
| Non-tumorous pancreas of individuals with tumour (PA34, PA35, PA36, PA37) | 4 | 4 | 0 | 0 |
| Non-tumorous inflamed pancreas of individuals with pancreatitis and tumour (PA28, PA29, PA30) | 3 | 3 | 0 | 0 |
| Pancreatic ductal adenocarcinoma stage IV. b (PA 23) | 1 | 0 | 1 | 1 |
| Pancreatic ductal adenocarcinoma Stage I (PA27, PA33) | 2 | 2 | 0 | 0 |
| Pulmonary metastasis of a cancer of the pancreas (PA39) | 1 | 1 | 0 | 0 |
| Non-tumorous lung of individuals with pulmonary metastasis (PA38) | 1 | 1 | 0 | 0 |
| Immortalised ductal cell lines (Capan-1, BxPC-3, PANC-1) | 3 | 0 | 3 | 3 |

## Claims

1. An *In vitro* method to detect the presence of a cancer of the pancreas in an individual, to determine the stage or severity of this cancer in an individual or to monitor the effect of the therapy administered to the individual with this cancer, that comprises:

   a) detection and/or quantification of the DDEF2 protein, of the mRNA of the ddef2 gene, or of the corresponding cDNA in a sample of the said individual, and
   b) comparison of the amount of DDEF2 protein, of the mRNA of the ddef2 gene or of the corresponding cDNA detected in a sample of an individual, with the respective amount of the DDEF2 protein, of the mRNA of the ddef2 gene, or of the corresponding cDNA detected in the samples of control individuals or in previous samples of the same individual or with normal reference values.

2. Method according to claim 1 in which the cancer of the pancreas is a ductal adenocarcinoma of the pancreas.

3. Method according to claim 1 in which this sample is a sample of pancreas tissue.

4. Method according to claim 3 in which this sample of pancreas tissue to be analysed is obtained by any conventional method, preferably by surgical resection.

5. Method according to claim 1 in which this sample is a sample of urine, blood, plasma, serum, gastric juice aspirate, bile or semen.

6. Method according to claim 1, in which the sample to be analysed is taken from an individual not previously diagnosed with cancer.

7. Method according to claim 1 in which the sample to be analysed is obtained from an individual who has been previously diagnosed with cancer, preferably in the pancreas.

8. Method according to claim 1 in which the sample to be analysed is obtained from an individual receiving treatment, or who has been treated previously against cancer, preferably of the pancreas.

9. Method according to claim 1 **characterised in that** it comprises the extraction of the sample, either to obtain an extract of proteins or an extract of total RNA.

10. Method according to claim 9 **characterised in that** the detection of the DDEF2 protein comprises a first step, in which the protein extract of the sample is placed in contact with a composition of one or more specific antibodies, against one or more epitopes of the DDEF2 protein and a second step, in which the complexes formed by the antibodies and the DDEF2 protein is quantified.

11. Method according to claim 10, **characterised in that** these antibodies correspond to monoclonal or polyclonal antibodies, intact or recombinant fragments of antibodies, combibodies and Fab or scFv antibody fragments, specific against the DDEF2 protein; these antibodies are human, humanised or of non-human origin.

12. Method according to claims 10 or 11 **characterised in that** in the quantification of the complexes formed by antibodies and the DDEF2 protein, the techniques used are selected from the group comprised by: western-blot, ELISA (Enzyme-Linked Immunosorbent assay), RIA (Radioimmunoassay), Competitive EIA (Competitive Enzyme Immunoassay), DAS-ELISA (Double Antibody Sandwich-Elisa), immunocytochemical or immunohistochemical techniques, techniques based on the use of biochips or protein microarrays that include specific antibodies, assays based on the precipitation of colloidal gold in formats such as *dipsticks;* or by affinity chromatography techniques, ligand binding assays or lectin binding assays.

**13.** Method according to claim 9 **characterised in that** the detection either of the mRNA or of the corresponding cDNA of the ddef2 gene, comprises a first step of amplification of the mRNA that is present in the extract of total RNA, or of the corresponding cDNA synthesised by reverse transcription of the mRNA; and a second quantification step of the amplification product from either the mRNA or the cDNA of the ddef2 gene.

**14.** Method according to claim 13 **characterised in that** the amplification is performed qualitatively or quantitatively, by RT-PCR using primer oligonucleotides, where the sequences of the primers used to amplify the sequence of the ddef2 gene are 5'-GCATGCCATTCTCCATCAGA -3' y 5'- CCACCCCTTAACAGATCACACA -3'.

**15.** Method according to claim 9 **characterised in that** the detection is done with specific probes either of mRNA or of the corresponding cDNA of the ddef2 gene, by techniques such as northern-blot or northern transfer.

**16.** Method according to claim 9 **characterised in that** the detection of the mRNA is done by Real time quantitative RT-PCR (Q-PCR).

**17.** Use of nucleotide or peptide sequences derived from the ddef2 gene, to detect *in vitro* the presence of a cancer of the pancreas, especially of a ductal adenocarcinoma of the pancreas, to determine *in vitro* the stage or severity of this cancer in the individual, or to monitor *in vitro* the effect of the therapy administered to an individual with this cancer.

**18.** *In vitro* method to identify and evaluate the efficacy of therapeutic compounds against cancer of the pancreas, especially against a ductal adenocarcinoma of the pancreas, that comprises:

a) placing in contact a culture of tumour cells with the candidate compound, in the appropriate conditions and for a suitable time for these to interact,
b) detecting and quantifying expression levels of the ddef2 gene or the DDEF2 protein or both, and
c) comparing those expression levels with those of the control cultures of tumour cells not treated with the candidate compound.

**19.** Use of the nucleotide or peptide sequence derived from the ddef2 gene, in methods to screen for, identify, develop and evaluate the efficiency of compounds to treat a cancer of the pancreas, especially a ductal adenocarcinoma of the pancreas.

**20.** An agent **characterised in that** it inhibits the expression and/or activity of the DDEF2 protein or **in that** it inhibits the carcinogenic effects of induction of expression of the DDEF2 protein.

**21.** An agent according to claim 19 selected from the group comprised by:

a) an antibody, or combination of antibodies, specific against one or more epitopes present in the DDEF2 protein, preferably a human or humanised monoclonal antibody; can also be a fragment of an antibody, a single chain antibody or an anti-idiotype antibody,
b) cytotoxic agents such as toxins, molecules with radioactive atoms or chemotherapeutic agents, including but not limited to small organic and inorganic molecules, peptides, phosphopeptides, antisense molecules, ribozymes, triple helix molecules, RNA interference *(small interfering RNA),* double stranded RNA etc., which inhibit expression and/or activity of the DDEF2 protein and
c) antagonistic compounds of the DDEF2 protein, which inhibit one or more of the functions of the DDEF2 protein.

**22.** Agent according to claims 19 or 20 to treat a cancer of the pancreas, especially a ductal adenocarcinoma of the pancreas.

**23.** Use of any of the agents according to claims 20 or 21 to elaborate a medicinal product to treat a cancer of the pancreas, especially a ductal adenocarcinoma of the pancreas.

**24.** Pharmaceutical composition that consists of a therapeutically effective amount of one or several agents according to claims 20 or 21 in combination with at least one pharmaceutically acceptable agent.

**25.** Pharmaceutical composition according to claim 24 characterised because it contains another active ingredient, preferably one that inhibits the function of the DDEF2 protein.

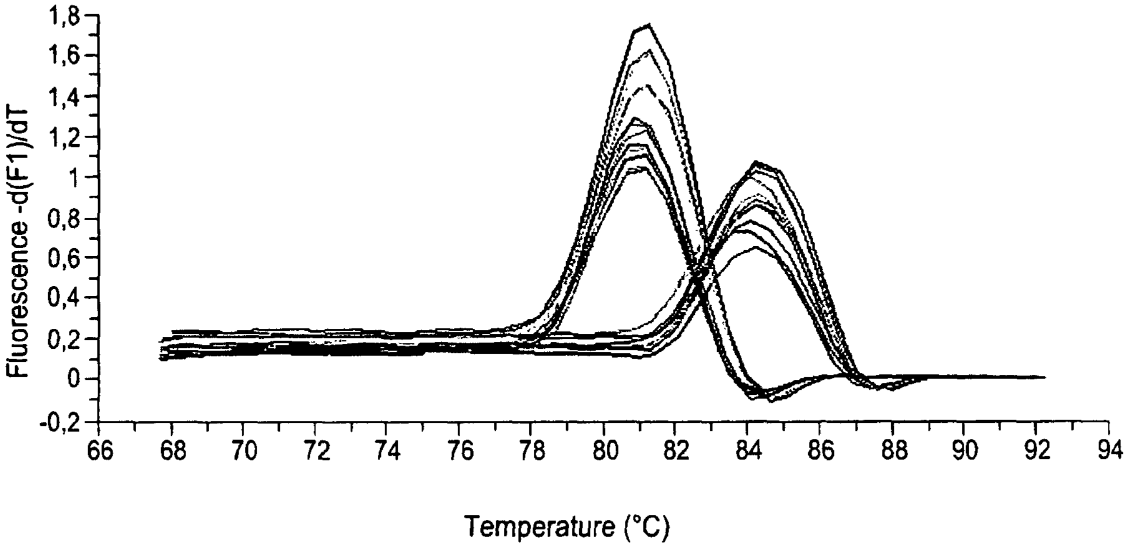
1,8
1,6
1,4
1,2
1
0,8
0,6
0,4
0,2
0
-0,2
Fluorescence -d(F1)/dT
66
68
70
72
74
76
78
80
82
84
86
88
90
92
94
Temperature (°C)

FIG.1

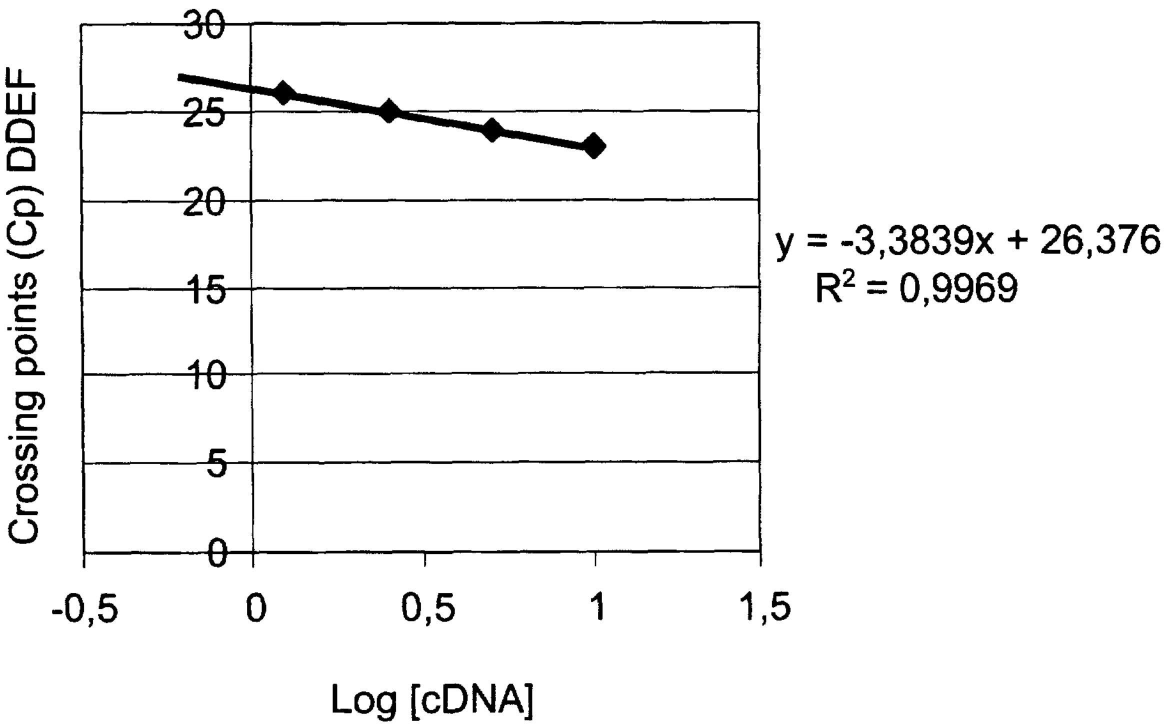

Crossing points (Cp) DDEF
30
25
20
15
10
5
0
-0,5
0
0,5
1
1,5
Log [cDNA]
y = -3,3839x + 26,376
R2 = 0,9969


FIG.2

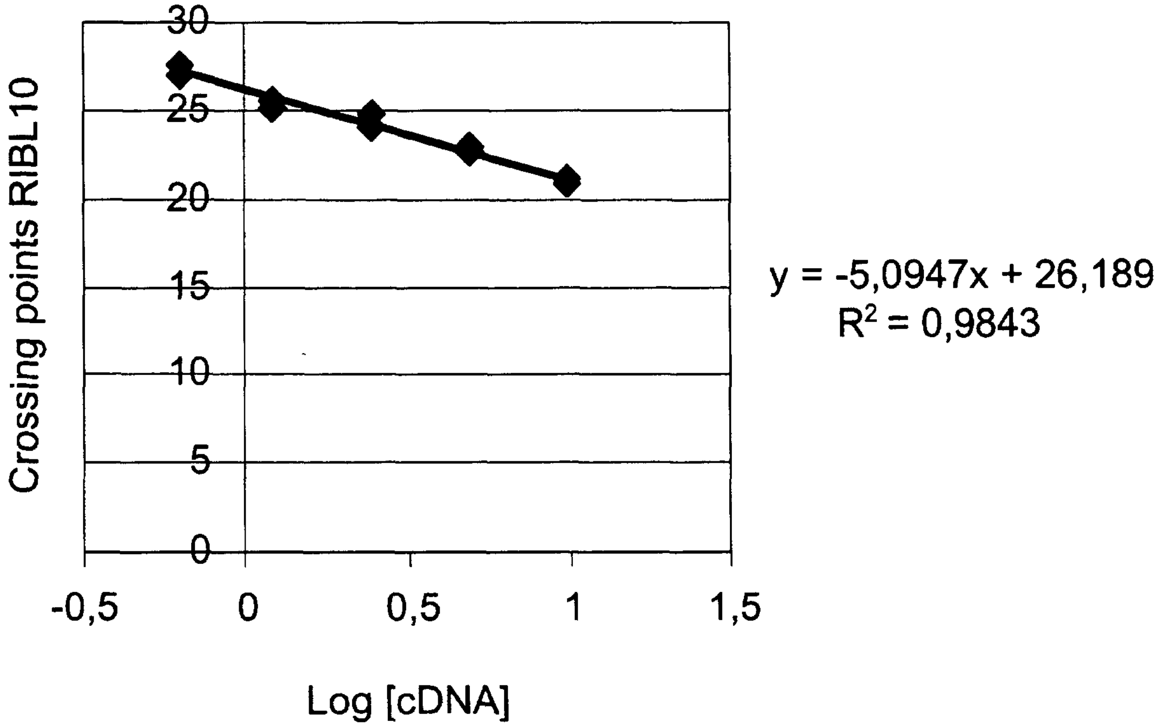
Crossing points RIBL10
30
25
20
15
10
5
0
-0,5
0
0,5
1
1,5
Log [cDNA]
y = -5,0947x + 26,189
R2 = 0,9843

FIG.3

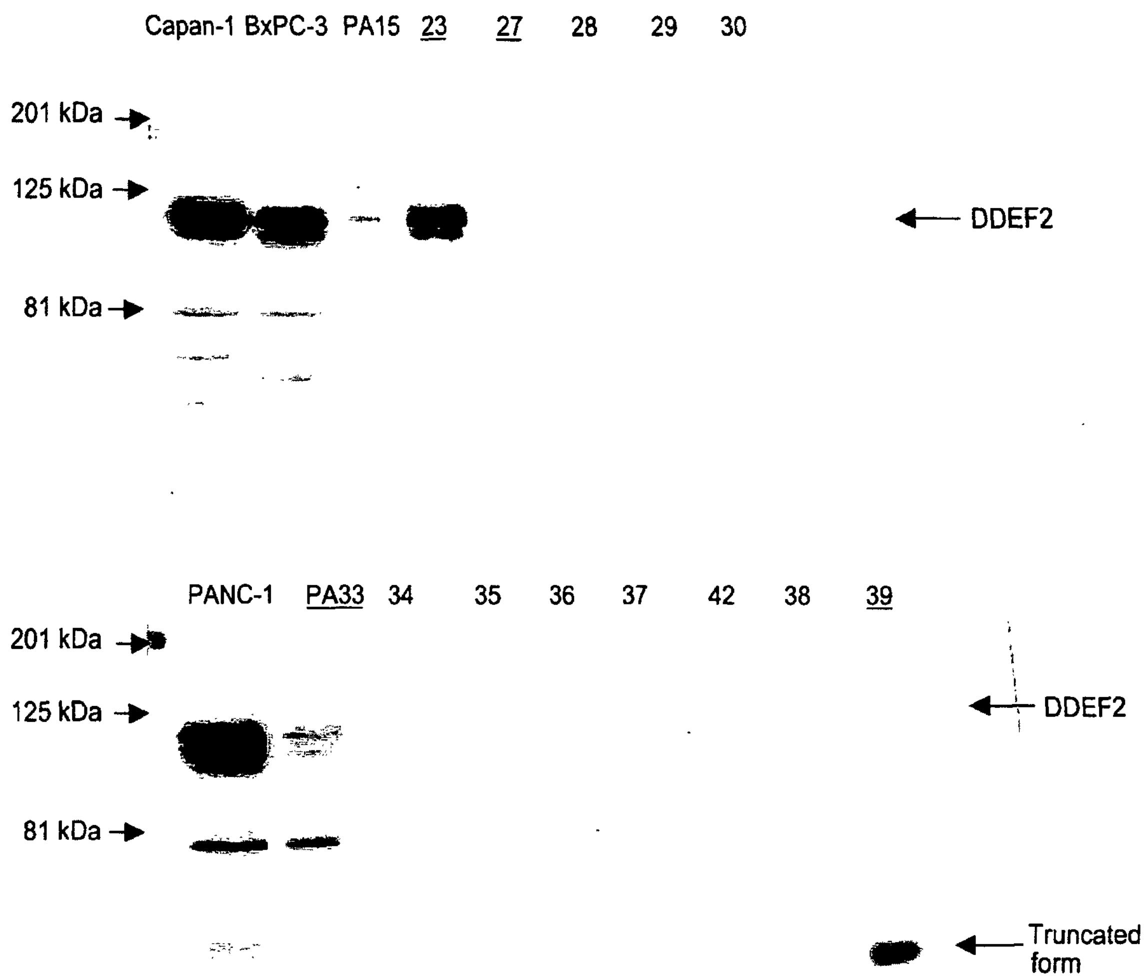
Capan-1 BxPC-3 PA15 23 27 28 29 30
201 kDa
125 kDa
81 kDa
DDEF2
PANC-1 PA33 34 35 36 37 42 38 39
201 kDa
125 kDa
81 kDa
DDEF2
Truncated form

FIG.4

European Patent Office

# PARTIAL EUROPEAN SEARCH REPORT

which under Rule 45 of the European Patent Convention shall be considered, for the purposes of subsequent proceedings, as the European search report

Application Number
EP 03 38 0238

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | ANDREEV, J. ET AL: "Identification of a new Pyk2 target protein with Arf-GAP activity" MOLECULAR AND CELLULAR BIOLOGY, vol. 19, no. 3, March 1999 (1999-03), pages 2338-2350, XP002273219 * the whole document * --- | 1-25 | G01N33/574 |
| A | KEOGH, R. J. ET AL: "Human endothelial Pyk2 is expressed in two isoforms and associates with paxilin and p130Cas" BIOCHEMICAL AND BIOPHYSICAL RESEARCH COMMUNICATIONS , vol. 290, 2002, pages 1470-1477, XP002273220 * abstract * --- -/-- | 1-25 | |

TECHNICAL FIELDS SEARCHED (Int.Cl.7)
G01N

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

1

| Place of search | Date of completion of the search | Examiner |
|---|---|---|
| MUNICH | 11 March 2004 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

EPO FORM 1503 03.82 (P04C07)

European Patent Office

# PARTIAL EUROPEAN SEARCH REPORT

Application Number
EP 03 38 0238

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| D,X | IACOBUZIO-DONAHUE C. A. ET AL: "Exploration of global gene expression patterns in pancreatic adenocarcinoma using cDNA microarrays" AMERCIAN JOURNAL OF PATHOLOGY, vol. 162, no. 4, April 2003 (2003-04), pages 1151-1152, XP009027111 * the whole document * ----- | 1-25 | |
| | | | TECHNICAL FIELDS SEARCHED (Int.Cl.7) |

1

EPO FORM 1503 03.82 (P04C10)

European Patent Office

INCOMPLETE SEARCH SHEET C

Application Number
EP 03 38 0238

Claim(s) searched incompletely:
20-25

Reason for the limitation of the search:

Present claims 20-25 relate to an extremely large number of possible compounds defined by their property of inhibiting the expresion or activity of the DDEF2 protein. Support within the meaning of Article 84 EPC and disclosure within the meaning of Article 83 EPC is to be found, however, for only a very small proportion of the compounds claimed. In the present case, the claims so lack support, and the application so lacks disclosure, that a meaningful search over the whole of the claimed scope is impossible. Consequently, the search has been carried out for those parts of the claims which appear to be supported and disclosed, namely the antibodies against the DDEF2 protein.